# EUROPEAN PATENT SPECIFICATION

(11) **EP 2 329 042 B1**
(45) Date of publication and mention of the grant of the patent: **26.10.2016**
(21) Application number: 09785026.7
(22) Date of filing: 01.09.2009
(51) Int. Cl.: C12Q 1/68, G01N 33/574

(54) **METHOD OF DIAGNOSING OR PROGNOSING EPITHELIAL OVARIAN CANCER**
VERFAHREN ZUR DIAGNOSE ODER PROGNOSE VON EPITHEL-OVARIALKARZINOM
PROCÉDÉ POUR ÉTABLIR LE DIAGNOSTIC OU LE PRONOSTIC D'UN CANCER ÉPITHÉLIAL DE L'OVAIRE

(30) Priority: 01.09.2008 GB 0815846
(43) Date of publication of application: 08.06.2011
(73) Proprietor: Immunovia AB, 223 81 Lund (SE)
(72) Inventor: BORREBAECK, Carl, Arne, Krister, S-223 63 Lund (SE); EK, Sara, Charlotte, Andersson, S-226 49 Lund (SE); BRENNAN, Donal, John, Portobello Dublin 8 (IE)
(74) Representative: Potter Clarkson LLP
(86) International application number: PCT/GB2009/002098
(87) International publication number: WO 2010/023458

(56) References cited:
- EP-A1- 1 806 413
- WO-A2-2005/002417
- WO-A2-2005/005661
- EK S ET AL: "Nuclear expression of the non B-cell lineage Sox11 transcription factor identifies mantle cell lymphoma" BLOOD CONF- 50TH ANNUAL MEETING OF THE AMERICAN- SOCIETY-OF-HEMATOLOGY; SAN FRANCISCO, CA, USA; DECEMBER 06 -09, 2008, AMERICAN SOCIETY OF HEMATOLOGY, vol. 111, no. 2, 12 October 2007 (2007-10-12), pages 800-805, XP007905438 ISSN: 0006-4971
- LEE CHING-JUNG ET AL: "Differential expression of SOX4 and SOX11 in medulloblastoma" JOURNAL OF NEURO-ONCOLOGY, KLUWER, BOSTON, US, vol. 57, no. 3, 1 May 2002 (2002-05-01), pages 201-214, XP002492273 ISSN: 0167-594X
- WEIGLE BERND ET AL: "Highly specific overexpression of the transcription factor SOX11 in human malignant gliomas" ONCOLOGY REPORTS, NATIONAL HELLENIC RESEARCH FOUNDATION, ATHENS, GR, vol. 13, no. 1, 1 January 2005 (2005-01-01), pages 139-144, XP009104552 ISSN: 1021-335X
- PANAGIOTIS A KONSTANTINOPOULOS ET AL: "Gene-expression profiling in epithelial ovarian cancer" NATURE CLINICAL PRACTICE ONCOLOGY, NATURE PUBLISHING GROUP, vol. 5, no. 10, 22 July 2008 (2008-07-22), pages 577-587, XP007910974 ISSN: 1743-4254
- SPENTZOS D ET AL: "Gene expression signature with independent prognostic significance in epithelial ovarian cancer" JOURNAL OF CLINICAL ONCOLOGY, AMERICAN SOCIETY OF CLINICAL ONCOLOGY, US, vol. 22, no. 23, 1 December 2004 (2004-12-01), pages 4700-4710, XP002382645 ISSN: 0732-183X
- TAMMELA J ET AL: "Gene expression and prognostic significance in ovarian cancer" MINERVA GINECOLOGICA, TORINO, IT, vol. 56, no. 6, 1 December 2004 (2004-12-01), pages 495-502, XP009126778 ISSN: 0026-4784
- BRENNAN DONAL J ET AL: "The transcription factor Sox11 is a prognostic factor for improved recurrence-free survival in epithelial ovarian cancer." EUROPEAN JOURNAL OF CANCER (OXFORD, ENGLAND : 1990) MAY 2009, vol. 45, no. 8, May 2009 (2009-05), pages 1510-1517, XP026056403 ISSN: 1879-0852

## Description

### Field of Invention

The present invention relates to novel agents for the diagnosis, prognosis and imaging of epithelial ovarian cancer (EOC), and use of the same.

### Introduction

Epithelial ovarian cancer (EOC) is the leading cause of death from gynaecological malignancy and the fifth most common cause of cancer related death in women. In 2008 it is estimated that 21,650 new ovarian cancer cases will be diagnosed in the United States and that 15,520 will succumb to the disease (Jemal A, Siegel R, Ward E, et a/. Cancer statistics, 2008. CA: a cancer journal for clinicians 2008;58:71-96). The poor ratio of survival to incidence in EOC is related to the high percentage of cases that are diagnosed at an advance stage and the lack of effective therapies for advanced refractory disease. Despite improvements in surgical techniques and the advent of more targeted therapeutics such as bevacizimab, survival of patients with EOC stands at 45% at five years (Jemal A, Siegel R, Ward E, et al. Cancer statistics, 2008. CA: a cancer journal for clinicians 2008;58:71-96). Such poor prognostic statistics indicate that there is an urgent need to improve our understanding of the molecular mechanisms underlying EOC, so as to develop better prognostic and predictive assays and identify new therapeutic targets.

Epithelial ovarian cancer comprises three major histological subtypes; serous, mucinous and endometrioid. Serous EOC includes serous cystomas, serous benign cystadenomas, serous cystadenomas with proliferating activity of the epithelial cells and nuclear abnormalities but with no infiltrative destructive growth (low potential or borderline malignancy), and serous cystadenocarcinomas. Mucinous EOC includes mucinous cystomas, mucinous benign cystadenomas, mucinous cystadenomas with proliferating activity of the epithelial cells and nuclear abnormalities but with no infiltrative destructive growth (low potential or borderline malignancy), and mucinous cystadenocarcinomas. Endometrioid EOC includes endometrioid tumours (similar to adenocarcinomas in the endometrium), endometrioid benign cysts, endometrioid tumours with proliferating activity of the epithelial cells and nuclear abnormalities but with no infiltrative destructive growth (low malignant potential or borderline malignancy), and endometrioid adenocarcinomas.

Two further, less-prevalent histological subtypes also exist, clear cell and undifferentiated.

In addition, EOC may be categorised by "stages", depending upon how far they have spread beyond the ovary. Thus, Stage I is defined as ovarian cancer that is confined to one or both ovaries. Stage II is defined as ovarian cancer that has spread to pelvic organs (e.g., uterus, fallopian tubes), but has not spread to abdominal organs. Stage III is defined as ovarian cancer that has spread to abdominal organs or the lymphatic system (e.g., pelvic or abdominal lymph nodes, on the liver, on the bowel). Finally, Stage IV is defined as ovarian cancer that has spread to distant sites (e.g., lung, inside the liver, brain, lymph nodes in the neck).

EOCs may also be graded according to the appearance of the cancer cells. Low-grade (or Grade 1) means that the cancer cells look very like the normal cells of the ovary; they usually grow slowly and are less likely to spread. Moderate-grade (or Grade 2) means that the cells look more abnormal than low-grade cells. High-grade (or Grade 3) means that the cells look very abnormal. They are likely to grow more quickly and are more likely to spread.

EOC, like most other cancers, is thus a complex heterogeneous disease, influenced and controlled by multiple genetic and epigenetic alterations leading to an increasingly aggressive phenotype (Martin L and Schilder R. Novel approaches in advancing the treatment of epithelial ovarian cancer: the role of angiogenesis inhibition. Journal of clinical oncology 2007;25:2894-901; Naora H and Montell DJ. Ovarian cancer metastasis: integrating insights from disparate model organisms. Nature reviews 2005;5:355-66). It is now well recognised that the characteristics of an individual tumour and its life course results from multiple somatic mutations acquired over time (e.g. TP53, PTEN, RAS) and continual evolution of the host responses to environmental factors (e.g., estrogen or tobacco exposure) (West M, Ginsburg G, Huang A, and Nevins J. Embracing the complexity of genomic data for personalized medicine. Genome Res 2006;16:559-66). In certain cases, these somatic mutations overlie inherent germline variations (e.g. BRCA1/2) (West M, Ginsburg G, Huang A, and Nevins J. Embracing the complexity of genomic data for personalized medicine. Genome Res 2006;16:559-66; Prat J, Ribe A, and Gallardo A. Hereditary ovarian cancer. Human pathology 2005;36:861-70). From a therapeutic standpoint EOC is best considered a collection of complex inter-related diseases represented by an immense natural heterogeneity in tumour phenotypes, disease outcomes, and response to treatment. A major challenge is consequently to identify and thoroughly validate diagnostic and prognostic biomarkers that can accurately describe the heterogeneity ascribed to EOC. In addition, accurate predictive biomarkers are required to guide current treatment protocols, as well as to guide the development and application of new targeted therapies.

Technologies, such as DNA microarrays, mass spectrometry-based proteomics and metabolomics have facilitated translational research over the last decade helping us to improve our understanding of the molecular and genetic basis of oncogenesis and by affording an opportunity to add new approaches to the practice of clinical oncology. The fundamental premise of "omic technologies" is that comprehensive examination of changes in the genome (DNA), transcriptome (mRNA), proteome (proteins), or metabolome (metabolites) can provide insight into the physiology and mechanism of disease, by the provision of superior diagnostic tests and therapeutic efficacy to that currently available (Brennan DJ, Kelly C, Rexhepaj E, et al. Contribution of DNA and Tissue Microarray Technology to the Identification and Validation of Biomarkers and Personalised Medicine in Breast Cancer. Cancer Genomics and Proteomics 2007;4:3-16). The application of DNA microarray technology to cancer biology, in particular, has led to an ever-growing comprehension of the complexity of the underlying pathophysiological pathways and interactions within a tumour (Duffy MJ, Kelly ZD, Culhane AC, O'Brien S, and Gallagher WM. DNA microarray-based gene expression profiling in cancer aiding cancer diagnosis, assessing prognosis and predicting response to therapy. Current Pharmacogenomics 2005;3:289-304; Brennan DJ, O'Brien SL, Fagan A, et al. Application of DNA microarray technology in determining breast cancer prognosis and therapeutic response. Expert Opin Biol Ther 2005;5:1069-83). Transcriptomic screens have accelerated research into genotypic-phenotypic correlations, with a common aim of elucidating the functional taxonomy of genes in both normal tissues and disease states, such as cancer (Brennan D, O'Brien S, Fagan A, et al. Application of DNA microarray technology in determining breast cancer prognosis and therapeutic response. Expert Opin Biol Ther 2005;5:1069-83).

Konstantinopoulos, P.A. et al., Nature Clinical Practice (2008), 5(10):577-587 published online 22 July 2008 and Spentzos, D. et al., Journal of Clinical Oncology (2004), 22(23):4700-4710 disclose gene expression profiles with prognostic significance in epithelial ovarian cancer.

However, there remains a need to improved agents and methods for the diagnosis and prognosis of EOC.

### Summary of Invention

The invention is defined in the appendant claims.

A first aspect of the disclosure relates to a binding moiety which is capable of binding selectively to Sox11 protein, or to a nucleic acid molecule encoding the same, for use in diagnosing or prognosing epithelial ovarian cancer (EOC).

It will be appreciated that the disclosure relates to provides the use of a binding moiety which is capable of binding selectively to Sox11 protein, or to a nucleic acid molecule encoding the same, in the preparation of a diagnostic or prognostic agent for epithelial ovarian cancer (EOC).

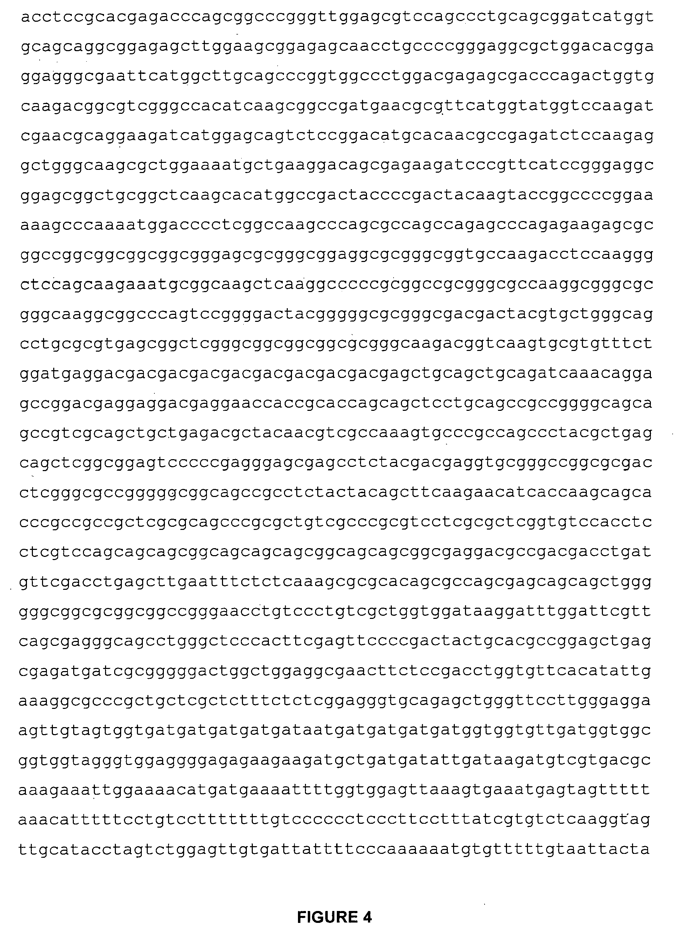

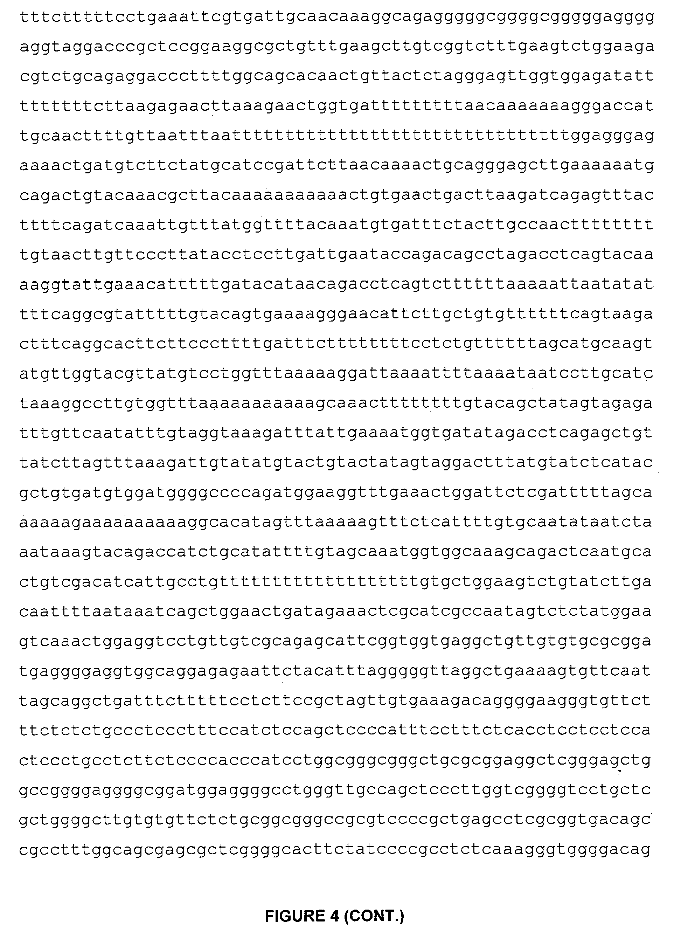

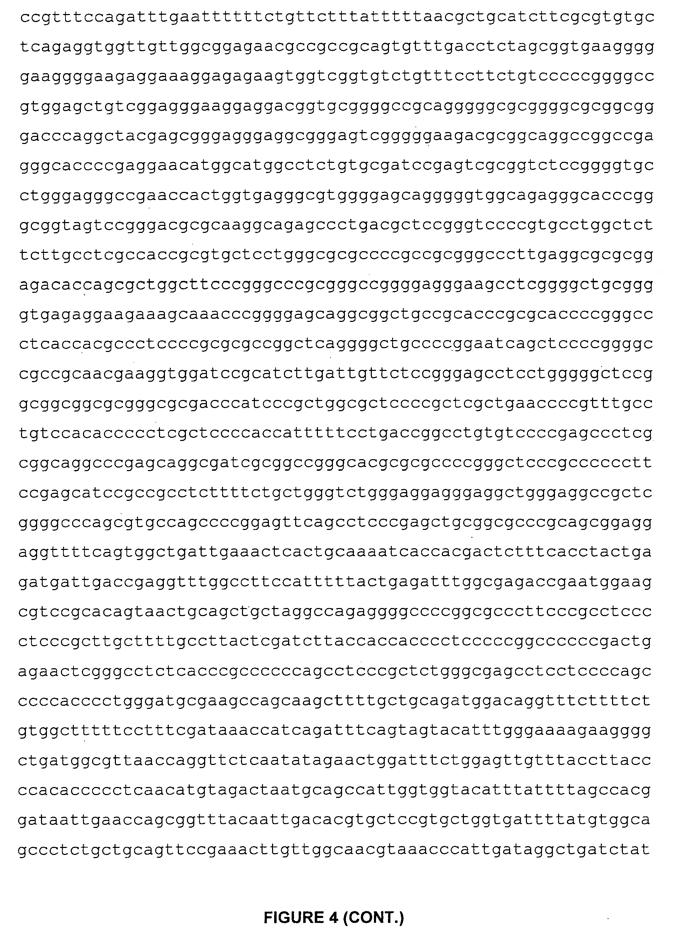

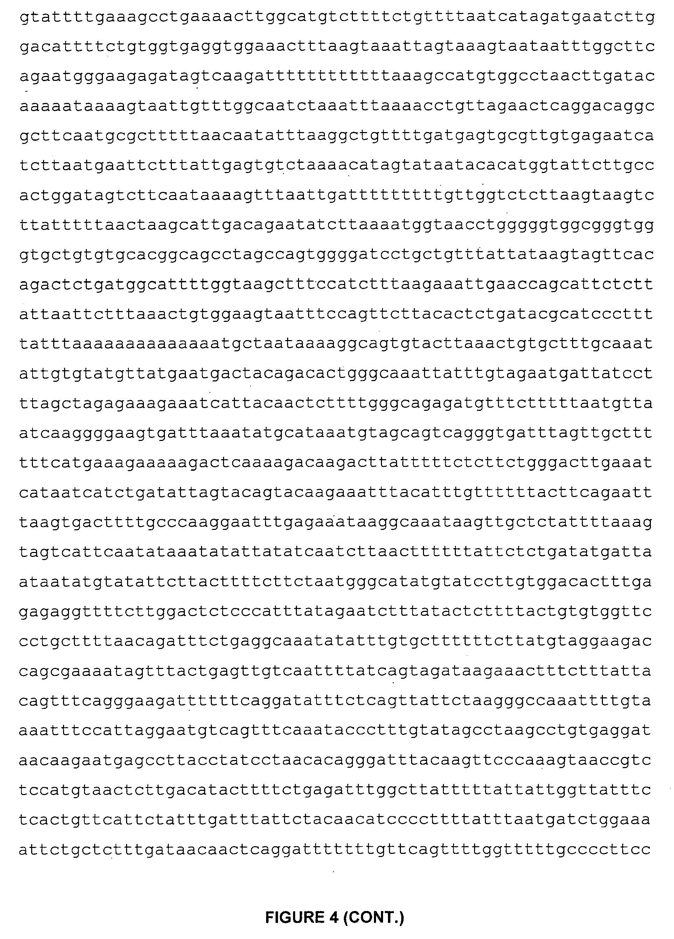

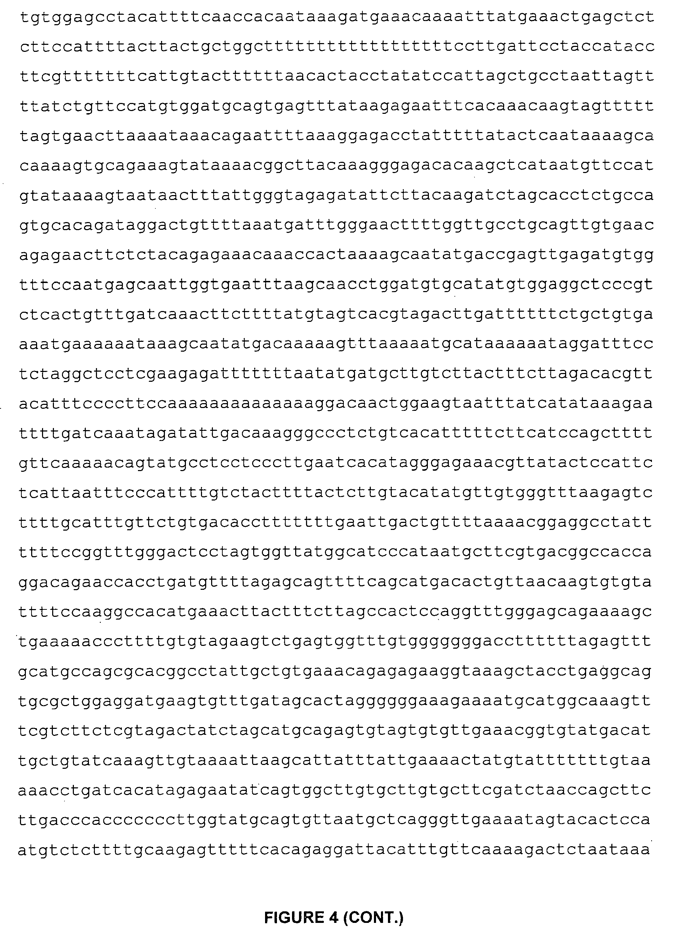

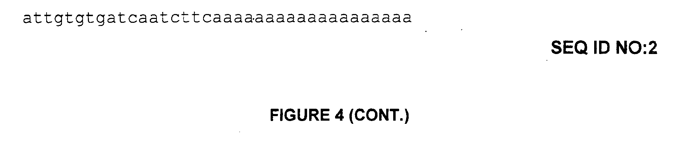

By "Sox11 protein" we include the amino acid sequence of the human Sox11 protein as shown in Figure 4 herein, as well as naturally-occurring homologues thereof.

Thus, we also include the Sox11 proteins as identified by Database Accession Nos. BAA88122, AAH25789, AAB08518, AAH25789 and P35716.

The present inventors have surprisingly identified Sox11 as a novel diagnostic/prognostic antigen for EOC, using immunohistochemistry analysis. Not only is this is the first report showing Sox11 overexpression in EOC cells but also the differential expression of Sox11 in high risk versus low risk EOC cohorts. Thus, Sox11 provides a valuable marker for diagnosing EOC patients and facilitates accurate diagnosis and/or prognosis of this aggressive malignancy.

By "diagnosing" we include the act or process of identifying the existence and/or type of cancer from which an individual may be suffering. Thus, in one embodiment, diagnosis includes the differentiation of a particular cancer type, namely EOC, from one or more other cancers. In an alternative embodiment, binding moieties of the disclosure are for use in classifying EOC patients into clinically relevant groups based on overall survival and/or cancer-specific survival.

By "prognosis" we include the act or process of predicting the probable course and outcome of a cancer, e.g. determining survival probability and/or recurrence-free survival (RFS) probability.

By "binding moiety" it is meant a molecule or entity which is capable of binding to Sox11 protein or mRNA encoding the same.

It will be appreciated by persons skilled in the art that the binding moieties of the disclosure may be used for the diagnosis or prognosis of EOC of any histological subtype (for example, serous, mucinous, endometrioid, clear cell, undifferentiated or unclassifiable).

In one embodiment, of the invention, the binding moiety is for use in diagnosing or prognosing EOC belonging to a specific histological subtype. Thus, the EOC may belong to a histological subtype selected from the group consisting of serous, mucinous, endometrioid, or clear cell.

Likewise, the binding moiety may be for use in diagnosing or prognosing EOC associated with cells of a specific grade (for example, high grade).

It will also be appreciated that the binding moieties of the disclosure may be used *in vivo* or *in vitro.*

In one embodiment, of the invention, the binding moiety of the invention is for use in the detection of Sox11 expression as a sole biomarker for epithelial ovarian cancer (EOC). For example, Sox11 expression may be used as a sole biomarker for the differentiation of EOC from one or more other cancers.

Alternatively, the binding moiety of the disclosure may be for use in combination with one or more additional binding moieties for detecting one or more additional biomarkers for epithelial ovarian cancer (EOC). Thus, the binding moiety may be for use in combination with fewer than 20 additional binding moieties, for example fewer than 15, 10, 8, 6, 5, 4, 3, 2 or 1 additional binding moieties.

In one particular embodiment, the binding moiety of the disclosure is for detecting nuclear and/or cytoplasmic expression of Sox11.

By "binding selectively" we include binding moieties which bind more strongly to Sox11 than to another polypeptides or nucleic acids; preferably at least 10-fold more strongly, more preferably at least 50-fold more strongly and even more preferably, at least 100-fold more strongly. Preferably, the binding moieties bind only to Sox11 polypeptides or nucleic acids.

The term 'polypeptide' as used herein means a plurality of amino acids that are linked together via a peptide bond. The term 'peptide' may be used interchangeably with the term 'polypeptide' however a peptide may be composed of two or more polypeptides.

The term 'amino acid' as used herein includes the standard twenty genetically-encoded amino acids and their corresponding stereoisomers in the 'D' form (as compared to the natural 'L' form), omega-amino acids other naturally-occurring amino acids, unconventional amino acids (*e*.*g*. α, α -disubstituted amino acids, N-alkyl amino acids, *etc.)* and chemically derivatised amino acids.

When an amino acid is being specifically enumerated, such as 'alanine' or 'Ala' or 'A', the term refers to both L-alanine and D-alanine unless explicitly stated otherwise. Other unconventional amino acids may also be suitable components for polypeptides of the present disclosure as long as the desired functional property is retained by the polypeptide. For the peptides shown, each encoded amino acid residue, where appropriate, is represented by a single letter designation, corresponding to the trivial name of the conventional amino acid.

Nucleic acid-based binding moieties of the disclosure are preferably DNA but may also be RNA or an artificial nucleic acid such as PNA.

The second aspect of the disclosure provides a binding moiety which is capable of binding selectively to Sox11 protein, or to a nucleic acid molecule encoding the same, for use in detecting epithelial ovarian cancer (EOC) cells in a sample.

In one embodiment, the binding moiety is for use in detecting EOC belonging to a specific histological subtype (e.g. serous, mucinous, endometrioid, clear cell and undifferentiated or unclassifiable).

Likewise, the binding moiety may be for use in detecting EOC associated with cells of a specific grade (e.g. high grade).

One embodiment of the disclosure provides a binding moiety according to either the first or second aspect which is capable of binding selectively to Sox11 protein. Preferably, the Sox11 protein is a human protein.

Conveniently, the binding moiety is capable of binding selectively to a polypeptide comprising an amino acid sequence of SEQ ID NO:1 (see figure 4) and/or a natural variant thereof.

By "natural variant" we include Sox11 proteins which are found in nature, for example allelic variants.

Variants of polypeptides include polypeptides comprising a sequence with at least 60% identity to known amino acid sequences, preferably at least 70% or 80% or 85% or 90% identity to said sequences, and more preferably at least 95%, 96%, 97%, 98% or 99% identity to said amino acid sequence of SEQ ID NO:1.

Percent identity can be determined by, for example, the LALIGN program (Huang and Miller, Adv. Appl. Math. (1991) 12:337-357) at the Expasy facility site (http://www.ch.embnet.org/software/LALIGN_form.html) using as parameters the global alignment option, scoring matrix BLOSUM62, opening gap penalty -14, extending gap penalty -4. Alternatively, the percent sequence identity between two polypeptides may be determined using suitable computer programs, for example the GAP program of the University of Wisconsin Genetic Computing Group and it will be appreciated that percent identity is calculated in relation to polypeptides whose sequence has been aligned optimally.

Preferably the binding moiety comprises or consists of a polypeptide.

Polypeptide binding moieties can be identified by means of a screen. A suitable method or screen for identifying peptides or other molecules which selectively bind a target protein or polypeptide may comprise contacting the target protein or polypeptide with a test peptide or other molecule under conditions where binding can occur, and then determining if the test molecule or peptide has bound the target protein or peptide. Methods of detecting binding between two moieties are well known in the art of biochemistry. Preferably, the known technique of phage display is used to identify peptides or other ligand molecules suitable for use as binding moieties. An alternative method includes the yeast two hybrid system.

More preferably the binding moiety comprises or consists of an antibody, or an antigen-binding fragment or variant thereof.

By "antibody" we include substantially intact antibody molecules, as well as chimaeric antibodies, humanised antibodies, human antibodies (wherein at least one amino acid is mutated relative to the naturally occurring human antibodies), single chain antibodies, bispecific antibodies, antibody heavy chains, antibody light chains, homodimers and heterodimers of antibody heavy and/or light chains, and antigen binding fragments and derivatives of the same.

By "antigen-binding fragment" we mean a functional fragment of an antibody that is capable of binding to Sox11 protein.

Preferably, the antigen-binding fragment is selected from the group consisting of Fv fragments (e.g. single chain Fv and disulphide-bonded Fv), Fab-like fragments (e.g. Fab fragments, Fab' fragments and F(ab)₂ fragments), single variable domains (e.g. V_{H} and V_{L} domains) and domain antibodies (dAbs, including single and dual formats [*i.e.* dAb-linker-dAb]).

The advantages of using antibody fragments, rather than whole antibodies, are several-fold. The smaller size of the fragments may lead to improved pharmacological properties, such as better penetration of solid tissue. Moreover, antigen-binding fragments such as Fab, Fv, ScFv and dAb antibody fragments can be expressed in and secreted from *E*. *coli,* thus allowing the facile production of large amounts of the said fragments.

Also included within the scope of the disclosure are modified versions of antibodies and an antigen-binding fragments thereof, e.g. modified by the covalent attachment of polyethylene glycol or other suitable polymer.

Methods of generating antibodies and antibody fragments are well known in the art. For example, antibodies may be generated via any one of several methods which employ induction of *in vivo* production of antibody molecules, screening of immunoglobulin libraries (Orlandi. et al, 1989. Proc. Natl. Acad. Sci. U.S.A. 86:3833-3837; Winter et al., 1991, Nature 349:293-299) or generation of monoclonal antibody molecules by cell lines in culture. These include, but are not limited to, the hybridoma technique, the human B-cell hybridoma technique, and the Epstein-Barr virus (EBV)-hybridoma technique (Kohler et al., 1975. Nature 256:4950497; Kozbor et al., 1985. J. Immunol. Methods 81:31-42; Cote et al., 1983. Proc. Natl. Acad. Sci. USA 80:2026-2030; Cole et al., 1984. Mol. Cell. Biol. 62:109-120).

Suitable monoclonal antibodies to selected antigens may be prepared by known techniques, for example those disclosed in *"*Monoclonal Antibodies: A manual of techniques", H Zola (CRC Press, 1988) and in *"*Monoclonal Hybridoma Antibodies: Techniques and Applications", J G R Hurrell (CRC Press, 1982).

Antibody fragments can be obtained using methods well known in the art (see, for example, Harlow & Lane, 1988, "Antibodies: A Laboratory Manual", Cold Spring Harbor Laboratory, New York). For example, antibody fragments according to the present disclosure can be prepared by proteolytic hydrolysis of the antibody or by expression in *E*. *coli* or mammalian cells (*e*.*g*. Chinese hamster ovary cell culture or other protein expression systems) of DNA encoding the fragment. Alternatively, antibody fragments can be obtained by pepsin or papain digestion of whole antibodies by conventional methods.

It will be appreciated by persons skilled in the art that for human therapy or diagnostics, humanised antibodies are preferably used. Humanised forms of non-human (e.g. murine) antibodies are genetically engineered chimaeric antibodies or antibody fragments having preferably minimal-portions derived from non-human antibodies. Humanised antibodies include antibodies in which complementary determining regions of a human antibody (recipient antibody) are replaced by residues from a complementary determining region of a non human species (donor antibody) such as mouse, rat of rabbit having the desired functionality. In some instances, Fv framework residues of the human antibody are replaced by corresponding non-human residues. Humanised antibodies may also comprise residues which are found neither in the recipient antibody nor in the imported complementarity determining region or framework sequences. In general, the humanised antibody will comprise substantially all of at least one, and typically two, variable domains, in which all or substantially all of the complementarity determining regions correspond to those of a non human antibody and all, or substantially all, of the framework regions correspond to those of a relevant human consensus sequence. Humanised antibodies optimally also include at least a portion of an antibody constant region, such as an Fc region, typically derived from a human antibody (see, for example, Jones et al., 1986. Nature 321:522-525; Riechmann et al., 1988, Nature 332:323-329; Presta, 1992, Curr. Op. Struct. Biol. 2:593-596).

Methods for humanising non-human antibodies are well known in the art. Generally, the humanised antibody has one or more amino acid residues introduced into it from a source which is non-human. These non-human amino acid residues, often referred to as imported residues, are typically taken from an imported variable domain. Humanisation can be essentially performed as described (see, for example, Jones et al., 1986, Nature 321:522-525; Reichmann et al., 1988. Nature 332:323-327; Verhoeyen et a/., 1988, Science 239:1534-1536I; US 4,816,567) by substituting human complementarity determining regions with corresponding rodent complementarity determining regions. Accordingly, such humanised antibodies are chimaeric antibodies, wherein substantially less than an intact human variable domain has been substituted by the corresponding sequence from a non-human species. In practice, humanised antibodies may be typically human antibodies in which some complementarity determining region residues and possibly some framework residues are substituted by residues from analogous sites in rodent antibodies.

Human antibodies can also be identified using various techniques known in the art, including phage display libraries (see, for example, Hoogenboom & Winter, 1991, J. Mol. Biol. 227:381; Marks et al., 1991, J. Mol. Biol, 222:581; Cole et al., 1985, In: Monoclonal antibodies and Cancer Therapy, Alan R. Liss, pp. 77; Boerner et al., 1991. J. Immunol. 147:86-95).

Once suitable antibodies are obtained, they may be tested for activity, for example by ELISA.

A further embodiment of the present disclosure provides a binding moiety capable of binding selectively to a nucleic acid molecule encoding Sox11 protein. Preferably, the binding moiety is capable of binding selectively to a nucleic acid molecule encoding a polypeptide comprising an amino acid sequence of SEQ ID NO:1 and/or natural variants thereof.

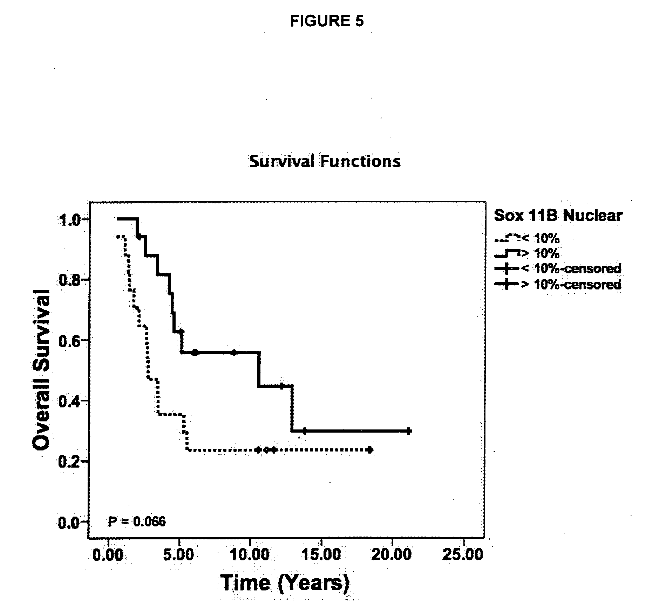

More preferably the binding moiety comprises or consists of a nucleic acid molecule. Even more preferably the binding moiety comprises or consists of a DNA molecule. Advantageously the binding moiety comprises or consists of a fragment of the nucleotide sequence of SEQ ID NO:2 (see figure 5), or the complementyary sequence thereof, of a fragment or a variant of the same. Conveniently, the nucleic acid molecule is 5 to 100 nucleotides in length. More conveniently the nucleic acid molecule is 15 to 35 nucleotides in length.

Preferably the binding moiety comprises a detectable moiety.

By a "detectable moiety" we include the meaning that the moiety is one which, when located at the target site following administration of the compound of the disclosure into a patient, may be detected, typically non-invasively from outside the body and the site of the target located. Thus, the compounds of this embodiment of the disclosure are useful in imaging and diagnosis.

Typically, the detectable moiety is or comprises a radioactive atom which is useful in imaging. Suitable radioactive atoms include ^{99m}Tc and ¹²³I for scintigraphic studies. Other readily detectable moieties include, for example, spin labels for magnetic resonance imaging (MRI) such as ¹²³I again, ¹³¹I, ¹¹¹In, ¹⁹F, ¹³C, ¹⁵N, ¹⁷O, gadolinium, manganese or iron. Clearly, the compound of the disclosure must have sufficient of the appropriate atomic isotopes in order for the molecule to be readily detectable.

The radio- or other labels may be incorporated in the compound of the disclosure in known ways. For example, if the binding moiety is a polypeptide it may be biosynthesised or may be synthesised by chemical amino acid synthesis using suitable amino acid precursors involving, for example, fluorine-19 in place of hydrogen. Labels such as ^{99m}Tc, ¹²³I, ¹⁸⁶Rh, ¹⁸⁸Rh and ¹¹¹In can, for example, be attached via cysteine residues in the binding moiety. Yttrium-90 can be attached via a lysine residue. The IODOGEN method (Fraker et al., 1978, Biochem. Biophys. Res. Comm. 80:49-57) can be used to incorporate ¹²³I. Reference ("Monoclonal Antibodies in Immunoscintigraphy", J-F Chatal, CRC Press, 1989) describes other methods in detail.

Thus, in a further embodiment of the disclosure the radioactive atom is selected from the group consisting of technetium-99m, iodine-123, iodine-125, iodine-131, indium-111, fluorine-19, carbon-13, nitrogen-15, oxygen-17, phosphorus-32, sulphur-35, deuterium, tritium, rhenium-186, rhenium-188 and yttrium-90.

A further aspect of the invention provides a method of diagnosing epithelial ovarian cancer (EOC) in an individual, the method comprising:
(a) providing a sample of epithelial ovarian cells from the individual; and
(b) determining the amount of Sox11 protein and/or mRNA in the sample of cells.
wherein the levels of Sox11 protein and/or mRNA are indicative of the individual having epithelial ovarian cancer (EOC).

In particular, high levels of Sox11 protein and/or mRNA are indicative of the individual having epithelial ovarian cancer (EOC).

In one embodiment, the method is for diagnosing EOC belonging to a specific histological subtype (*e*.*g*. serous, mucinous, endometrioid, and clear cell).

Likewise, the method may be for diagnosing EOC associated with cells of a specific grade (e.g. high grade).

In one embodiment, the method further comprises conducting one or more additional diagnostic tests for EOC on the epithelial ovarian cells to confirm the diagnosis

A further aspect of the invention provides a method of prognosing epithelial ovarian cancer (EOC) in an individual, the method comprising:
(a) providing a sample of epithelial ovarian cancer cells from the individual; and (b) determining the amount of Sox11 protein and/or mRNA in the sample of cells.
wherein the levels of Sox11 protein and/or mRNA are indicative of the chance of recurrence-free survival of the individual.

In one embodiment, the method is for prognosing EOC belonging to a specific histological subtype (*e*.*g*. serous, mucinous, endometrioid, and clear cell).

Likewise, the method may be for prognosing EOC associated with cells of a specific grade (e.g. high grade).

By "chance of recurrence-free survival" we mean the probability of the individual surviving for a given period, such as one year, two years, three years, five years, ten years or more.

In one embodiment, high levels of Sox11 protein and/or mRNA is indicative of the individual having improved recurrence-free survival (RFS).

By "improved recurrence-free survival" we mean that the probability of recurrence-free survival is higher when compared to an average population of epithelial ovarian cancer (EOC) patients, for example the probability may be increased by at least 0.05, 0.1, 0.2, 0.3, 0.4 or 0.5 or more.

In an alternative embodiment, low levels of Sox11 protein and/or mRNA is indicative of the individual having diminished recurrence-free survival (RFS).

By "diminished recurrence-free survival" we mean that the probability of recurrence-free survival is lower when compared to an average population of epithelial ovarian cancer (EOC) patients, for example the probability may be lowered by at least 0.05, 0.1, 0.2, 0.3, 0.4 or 0.5 or more.

A further aspect of the invention provides a method of detecting an epithelial ovarian cancer (EOC) in an individual, the method comprising:
(a) providing a sample of cells from the individual; and
(b) determining the amount of Sox11 protein and/or mRNA in the sample of cells.
wherein the levels of Sox11 protein and/or mRNA are indicative of the individual having epithelial ovarian cancer (EOC) cells.

In one embodiment, the method is for detecting cells of an EOC belonging to a specific histological subtype (*e*.*g*. serous, mucinous, endometrioid, and clear cell).

Likewise, the method may be for detecting cells of an EOC associated with cells of a specific grade (*e.g*. high grade).

In particular, high levels of Sox11 protein and/or mRNA are indicative of the individual having epithelial ovarian cancer (EOC).

By "high levels of Sox11 protein and/or mRNA" we mean the amount of Sox11 protein and/or mRNA is at least 10% higher than in non-cancerous (*e*.*g*. healthy)_epithelial ovarian cells, for example 20%, 30%, 40%, 50%, 60%, 70%, 80%, 90%, 100%, 120%, 150%, 200%, 300%, 400%, 500%, 600%, 700%, 800%, 900%, 1000%, 1500%, 2000%, 3000%, 4000%, 5000%, 10000% higher, or more.

By "low levels of Sox11 protein and/or mRNA" we mean the amount of Sox11 protein and/or mRNA is not statistically different from that of non-cancerous epithelial ovarian cells.

It will be appreciated by skilled persons that the methods of the invention may be performed *in vivo* or *in vitro.*

In one embodiment, the method comprises the detection of Sox11 expression as a sole biomarker for the diagnosis or prognosis of epithelial ovarian cancer (EOC) (as discussed above).

Alternatively, the binding moiety of the disclosure may be used in combination with one or more additional binding moieties for detecting one or more additional biomarkers for the diagnosis or prognosis of EOC. Thus, the binding moiety may be used in combination with fewer than 20 additional binding moieties, for example fewer than 15, 10, 8, 6, 5, 4, 3, 2 or 1 additional binding moieties.

In one embodiment, the method comprises detecting nuclear and/or cytoplasmic expression of Sox11.

Preferably the sample of cells to be tested is in the form of a tissue sample. Conveniently, the amount of Sox11 protein and/or mRNA in the sample is determined using a binding moiety according to the first or second aspect of the disclosure.

A further embodiment of the methods of the invention comprises comparing the amount of Sox11 protein and/or mRNA in the sample of cells to be tested with the amount of Sox11 protein and/or mRNA in a control sample.

Advantageously the control sample is a negative control sample comprising or consisting of non-cancerous epithelial ovarian cells.

Alternatively, or in addition, the control sample is a positive control sample comprising or consisting of epithelial ovarian cancer cells. The ovarian epithelial cells may be high recurrence-free survival (RFS)-associated EOC cells or low recurrence-free survival (RFS)-associated EOC cells.

Typically, step (b) of the methods of the invention is performed using a method selected from the group consisting of macroarray, microarray(including tissue microarray), nanoarray, reverse transcription PCR, real-time PCR or *in situ* PCR.

A further embodiment of the third or fourth aspects of the invention comprises determining the levels of additional EOC biomarker proteins and/or mRNA in the sample to cells to be tested, for example p53, estrogen receptor and/or progesterone receptor.

A further aspect of the invention provides provides a binding moiety for use in a method of imaging epithelial ovarian cancer (EOC) cells in the body of an individual, the method comprising administering to the individual an effective amount of a binding moiety according to the first or second aspect of the disclosure.

In one embodiment, the method is for imaging cells of an EOC belonging to a specific histological subtype (*e*.*g*. serous, mucinous, endometrioid, and clear cell).

Likewise, the method may be for imaging cells of an EOC associated with cells of a specific grade (*e*.*g*. high grade).

The term 'effective amount' as used herein, refers to that amount which provides a sufficiently detectable signal for a given administration regimen. This is a predetermined quantity of active material calculated to produce a desired signal strength in association with the required additive and diluent, *i.e.* a carrier or administration vehicle. As is appreciated by those skilled in the art, the amount of a compound may vary depending on its specific activity. Suitable dosage amounts may contain a predetermined quantity of active composition calculated to produce the desired signal strength in association with the required diluent. In the methods and use for manufacture of compositions of the disclosure, an effective amount of the active component is provided. An effective amount can be determined by the ordinary skilled medical or veterinary worker based on patient characteristics, such as age, weight, sex, condition, complications, other diseases, *etc.,* as is well known in the art.

Typically, the sixth aspect of the invention comprises the step of detecting the location of the binding moiety in the individual. Preferably Sox11 protein and/or mRNA encoding the same are used as a marker for epithelial ovarian cancer (EOC) cells.

A seventh aspect of the disclosure provides the use of a binding moiety as defined above in the preparation of a medicament for diagnosing or prognosing epithelial ovarian cancer (EOC).

The disclosure additionally relates to the use of Sox11 protein and/or mRNA encoding the same as a biomarker for epithelial ovarian cancer (EOC) cells.

In one embodiment, the Sox11 protein and/or mRNA encoding the same is for use as a biomarker for EOC belonging to a specific histological subtype (e.g. serous, mucinous, endometrioid, clear cell and undifferentiated or unclassifiable).

Likewise, the Sox11 protein and/or mRNA encoding the same may be for use as a biomarker for EOC belonging to a specific grade (e.g. high grade).

It will be appreciated that Sox11 may be used as a sole biomarker for epithelial ovarian cancer (EOC).

Alternatively, Sox11 may be used in combination with one or more additional biomarkers. Preferably, however, Sox11 is used in combination with fewer than 20 additional biomarkers are used in the method, for example fewer than 15, 10, 8, 6, 5, 4, 3, 2 or 1 additional biomarkers.

A ninth aspect of the disclosure additionally relates to a method of screening for a molecule with efficacy in the diagnosis and/or prognosis of epithelial ovarian cancer (EOC), the method comprising the steps of:
(a) contacting a molecule to be tested with Sox11 protein and/or mRNA encoding the same (or with a fragment of said protein or mRNA); and
(b) detecting the presence of a complex containing the protein and/or mRNA (or fragment thereof) and the molecule to be tested.

In one embodiment, the method is for screening for a molecule with efficacy in the diagnosis and/or prognosis of EOC belonging to a specific histological subtype (e.g. serous, mucinous, endometrioid).

Likewise, the method may be for screening for a molecule with efficacy in the diagnosis and/or prognosis of EOC associated with cells of a specific grade (e.g. high grade). Methods of detecting and/or measuring the concentration of protein and/or nucleic acid are well known to those skilled in the art, see for example Sambrook and Russell (*supra.).*

Preferred methods for detection and/or measurement of protein include Western blot, North-Western blot, immunosorbent assays (ELISA), antibody microarray, tissue microarray (TMA), immunoprecipitation, *in situ* hybridisation and other immunohistochemistry techniques, radioimmunoassay (RIA), immunoradiometric assays (IRMA) and immunoenzymatic assays (IEMA), including sandwich assays using monoclonal and/or polyclonal antibodies. Exemplary sandwich assays are described by David *et al.,* in US Patent Nos. 4,376,110 and 4,486,530, hereby incorporated by reference. Antibody staining of cells on slides may be used in methods well known in cytology laboratory diagnostic tests, as well known to those skilled in the art.

Typically, ELISA involves the use of enzymes which give a coloured reaction product, usually in solid phase assays. Enzymes such as horseradish peroxidase and phosphatase have been widely employed. A way of amplifying the phosphatase reaction is to use NADP as a substrate to generate NAD which now acts as a coenzyme for a second enzyme system. Pyrophosphatase from *Escherichia coli* provides a good conjugate because the enzyme is not present in tissues, is stable and gives a good reaction colour. Chemi-luminescent systems based on enzymes such as luciferase can also be used.

Conjugation with the vitamin biotin is frequently used since this can readily be detected by its reaction with enzyme-linked avidin or streptavidin to which it binds with great specificity and affinity.

Preferred methods for detection and/or measurement of nucleic acid (e.g. mRNA) include southern blot, northern blot, polymerase chain reaction (PCR), reverse transcriptase PCR (RT-PCR), quantitative real-time PCR (qRT-PCR), nanoarray, macroarray, autoradiography and *in situ* hybridisation.

In a typical embodiment the presence of epithelial ovarian cancer (EOC) cells is detected by detection of Sox11 protein and/or nucleic acid in cell nuclei and/or cytoplasm. Preferably, the nuclei/cytoplasm of lymphoma cells express Sox11 protein and/or nucleic acid in a relatively high amount, as indicated, for example, by bright staining of the nuclei during *in situ* hybridisation analysis.

Preferred, non-limiting examples which embody certain aspects of the invention will now be described, with reference to the following figures:
**Figure 1****. Sox11 protein expression in ovarian cancer.**
   Immunohistochemical staining of Sox 11 showing just cytoplasmic expression (A) and cytoplasmic and nuclear expression (B). Corresponding markup image of the deconvolution algorithm showing stroma in blue, cytoplasmic staining in yellow and orange and nuclear expression in red.
**Figure 2****. Sox11 protein expression and survival in ovarian cancer.**
   Sox11 expression classified into low, medium and high based on the histogram (A). Kaplan Meier estimate of RFS based on the three Sox11 groups (B). Kaplan Meier estimate of RFS based on comparison of high and medium levels of Sox11 to low levels of Sox11 (C).

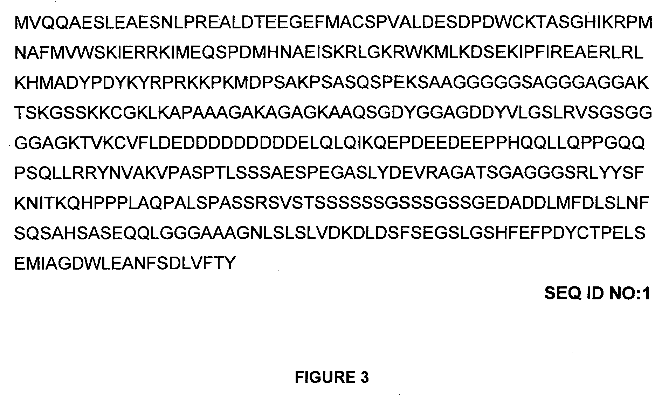

**Figure 3****. Amino acid sequence of *Homo sapiens* Sox11 protein.**
**Figure 4****. Nucleic acid sequence of *Homo sapiens SOX11* mRNA.**
**Figure 5****. Overall survival (25 years) in endometroid ovarian cancer**
   The 25-year overall survival of patients with more or less than 10% Sox11 positive tumor cells are compared. When a patient is censored (removed from the study for other reason than death) this is indicated with a tick-mark on the line.
**Figure 6****. Overall survival (5 years) in endometroid ovarian cancer**
   The 5-year overall survival of patients with more or less than 10% Sox11 positive tumor cells are compared. When a patient is censored (removed from the study for other reason than death) this is indicated with a tick-mark on the line.
**Figure 7****. Cancer specific survival (5 years) in endometroid ovarian cancer**
   The 5-year cancer-specific survival of patients with more or less than 10% Sox11 positive tumor cells are compared. When a patient is censored (removed from the study for other reason than death) this is indicated with a tick-mark on the line.
**Figure 8****. Overall survival (25 years) for high grade EOC**
   The 25-year overall survival of high-grade patients with more or less than 10% Sox11 positive tumor cells are compared. When a patient is censored (removed from the study for other reason than death) this is indicated with a tick-mark on the line.
**Figure 9****. Cancer specific (25 years) survival for high grade EOC**
   The 25-year cancer-specific survival of high-grade patients with more or less than 10% Sox11 positive tumor cells are compared. When a patient is censored (removed from the study for other reason than death) this is indicated with a tick-mark on the line.

### EXAMPLE A

### Introduction

The transcription factor Sox11 is a member of the Sox gene family and has been mapped to chromosome 2p25.3 (Azuma T, Ao S, Saito Y, et al. Human SOX11, an upregulated gene during the neural differentiation, has a long 3' untranslated region. DNA research 1999;6:357-60). Sox proteins are identified as proteins that contain a DNA-binding high mobility group (HMG) domain with strong amino acid homology (usually >50%) to the HMG domain of the male sex determination gene, Sry (Wegner M. From head to toes: the multiple facets of Sox proteins. Nucleic acids research 1999;27:1409-20). More than 20 orthologous Sox genes have been identified in the human and mouse genomes, and family members are divided into eight subgroups according to the degree of homology within and outside the HMG-domain (Schepers GE, Teasdale RD, and Koopman P. Twenty pairs of sox: extent, homology, and nomenclature of the mouse and human sox transcription factor gene families. Developmental cell 2002;3:167-70). Sox proteins act as transcription factors by binding to the minor groove of DNA and inducing a sharp bend of DNA allowing them to play a key architectural role in the assembly of transcriptional enhancer complexes (Dy P, Penzo-Mendez A, Wang H, et al. The three SoxC proteins Sox4, Sox11 and Sox12 exhibit overlapping expression patterns and molecular properties. Nucleic acids research 2008;36:3101-17; van de Wetering M and Clevers H. Sequence-specific interaction of the HMG box proteins TCF-1 and SRY occurs within the minor groove of a Watson-Crick double helix. The EMBO journal 1992;11:3039-44). In addition to protein-DNA interactions, Sox proteins also interact with various other transcription factors to increase their efficiency and specificity of action (Dy P, Penzo-Mendez A, Wang H, et al. The three SoxC proteins Sox4, Sox11 and Sox12 exhibit overlapping expression patterns and molecular properties. Nucleic acids research 2008;36:3101-17).

Sox11 belongs to the C subgroup, along with Sox4 and Sox12 (Schepers GE, Teasdale RD, and Koopman P. Twenty pairs of sox: extent, homology, and nomenclature of the mouse and human sox transcription factor gene families. Developmental cell 2002;3:167-70), and all three proteins demonstrate a high degree of homology within both the C-terminal transactivation domain and the HMG domain (Dy P, Penzo-Mendez A, Wang H, et al. The three SoxC proteins Sox4, Sox11 and Sox12 exhibit overlapping expression patterns and molecular properties. Nucleic acids research 2008;36:3101-17; Jay P, Goze C, Marsollier C, et al. The human SOX11 gene: cloning, chromosomal assignment and tissue expression. Genomics 1995;29:541-5). Sox11 and Sox 4 play major roles in cardiac, neuronal and other major embryonic processes, whilst less is known about Sox12 (Dy P, Penzo-Mendez A, Wang H, et al. The three SoxC proteins Sox4, Sox11 and Sox12 exhibit overlapping expression patterns and molecular properties. Nucleic acids research 2008;36:3101-17).

We have recently demonstrated the nuclear Sox11 is specifically up-regulated in mantle cell lymphoma (MCL) and distinguishes MCL from other B-cell lymphomas (Ek S, Dictor M, Jerkeman M, Jirstrom K, and Borrebaeck CA. Nuclear expression of the non B cell lineage Sox11 transcription factor identifies mantle cell lymphoma. Blood 2008;111:800-5). Sox4 is a prominent transcription factor in lymphocytes of both the B and T-cell lineage (Wegner M. From head to toes: the multiple facets of Sox proteins. Nucleic acids research 1999;27:1409-20; van de Wetering M, Oosterwegel M, van Norren K, and Clevers H. Sox-4, an Sry-like HMG box protein, is a transcriptional activator in lymphocytes. The EMBO journal 1993;12:3847-54) and is crucial for B lymphopoiesis (Smith E and Sigvardsson M. The roles of transcription factors in B lymphocyte commitment, development, and transformation. Journal of leukocyte biology 2004;75:973-81), whilst Sox11 has no known lymphopoietic function and is not expressed in B cells (Ek S, Dictor M, Jerkeman M, Jirstrom K, and Borrebaeck CA. Nuclear expression of the non B-cell lineage Sox11 transcription factor identifies mantle cell lymphoma. Blood 2008;111:800-5). Both Sox4 and Sox11 are expressed in medulloblastoma (Lee CJ, Appleby VJ, Orme AT, Chan WI, and Scotting PJ. Differential expression of SOX4 and SOX11 in medulloblastoma. Journal of neuro-oncology 2002;57:201-14) and Sox11 is also overexpressed in malignant glioma (Weigle B, Ebner R, Temme A, et al. Highly specific overexpression of the transcription factor SOX11 in human malignant gliomas. Oncology reports 2005;13:139-44). Additionally Sox4 is expressed in bladder cancer with increased levels of expression associated with improved patient outcome (Aaboe M, Birkenkamp-Demtroder K, Wiuf C, et al. SOX4 expression in bladder carcinoma: clinical aspects and in vitro functional characterization. Cancer research 2006;66:3434-42).

This study outlines the expression of Sox11 mRNA across a large number of normal tissues and tumours, and reveals Sox11 mRNA to be overexpressed in a large number of malignant tissues. In addition, we specifically examined Sox11 protein expression in EOC and demonstrated that increased levels of Sox11 protein, as determined by image analysis, were associated with an improve recurrence free survival (RFS).

### Materials and Methods

### Transcriptional profiling

SOX11 gene mRNA expression levels across a large number of human tissues were retrieved from the In Silico Transcriptomics (IST) database, containing data from a meta-analysis of 14,095 samples analyzed using the Affymetrix gene expression microarrays.

### Patients and tumour samples

The TMA, used in this study, was constructed from a consecutive cohort of 76 patients diagnosed with primary invasive epithelial ovarian cancer at the National Maternity Hospital, Dublin, with a median follow-up of of 4.3 years. The patient cohort is summarised in table 1. The standard surgical approach was a total abdominal hysterectomy, bilateral salpingo-oophorectomy and omentectomy with cytological evaluation of peritonea fluid or washings. Residual disease was resected to less than 2 cm where possible. Stage and volume of residual disease (no residual disease, residual disease greater or less than 2 cm) was recorded in all cases. Adjuvant chemotherapy consisted of cisplatin or carboplatin prior to 1992 and combined with paclitaxel from 1992 to 2002. No patient received neo-adjuvant chemotherapy. Benign or borderline ovarian cancers, non-epithelial ovarian cancer and cases with histological features typical of secondary ovarian cancer were excluded from the study. Diagnostic specimens were all formalin fixed and paraffin embedded in the Department of Pathology at the National Maternity Hospital, Dublin, Ireland. All tissue blocks were stored in this department prior to construction of the TMA. Full ethical approval was obtained from the ethics committee of the National Maternity Hospital, Dublin.

### Tissue microarrays and immunohistochemistry

Seventy six paraffin-embedded tumour specimens were used for tissue microarray (TMA) construction. Areas representative of invasive cancer were marked on haematoxylin and eosin-stained slides and the TMA was constructed, using a manual tissue arrayer (MTA-1, Beecher Inc, WI). The array consisted of four cores per patient. Two 1.0 mm cores were extracted from each donor block and assembled in a recipient block. Recipient blocks were limited to approximately 100 cores each. In general, cores were taken from the peripheral part of the tumour in cases where the tumour had well-defined borders. In more diffusely growing tumours, areas with the highest tumour cell density were primarily targeted. Necrotic tissue was avoided.

TMA sections (4 µm) were dried, deparaffinized, rehydrated and put through descending concentrations of ethanol. Heat mediated antigen retrieval was performed in a BORGdecloaker (Biocare, Concord, CA, USA) at pH 9.0 and sections were then stained with the primary rabbit anti-human Sox11 antibody (1:100) at room temperature for 25 minutes. This specific antibody was raised, as previously described (Ek S, Dictor M, Jerkeman M, Jirstrom K, and Borrebaeck CA. Nuclear expression of the non B-cell lineage Sox11 transcription factor identifies mantle cell lymphoma. Blood 2008; 111:800-5) and targeted the following protein sequence:

FMVWSKIERRKIMEQSPDMHNAEISKRLGKRWKMLKDSEKIPFIREAERLRLKHMADYP
DYKYRPRKKPKMDPSAKPSASQSPEKSAAGGGGGSAGGGAGGAKTSKGSSKK
[SEQ ID NO:3]

Signal was detected, using the Dako REAL Detection system, containing the secondary biotinylated goat anti-rabbit/mouse antibody, the strepavidin/horseradish peroxidase complex and 3,3 - diaminobenzidine, according to the manufacturer's protocol. Slides were counterstained with Mayers hematoxylin (Sigma-Aldrich, St Louis, MO).

### Image Acquisition, Management and Automated analysis

The Aperio ScanScope XT Slide Scanner (Aperio Technologies, Vista, CA) system was used to capture whole slide digital images with a 20X objective. Slides were de-arrayed to visualise individual cores, using TMA Lab (Aperio). A color deconvolution algorithm (Aperio) was used to develop a quantitative scoring model for Sox11 expression.

### Statistical analysis

Spearman's Rho correlation was used estimate the relationship between cores from individual tumours, Differences in distribution of clinical data and tumour characteristics between samples with a high and low Sox11 expression (described below) were evaluated using the χ² test. Kaplan-Meier analysis and the log rank test were used to illustrate differences between RFS. Cox regression proportional hazards models were used to estimate the relationship to RFS and Sox11, stage and grade. All calculations were performed, using SPSS version 11.0 (SPSS Inc, Chicago, IL). P value < 0.05 was considered statistically significant.

### Results

### Sox11 mRNA expression in normal and tumour tissues

A metanalysis of Sox11 mRNA expression levels was performed in 14 095 samples analyzed using the Affymetrix gene expression microarrays.

Increased levels of Sox11 mRNA expression were evident in epithelial ovarian carcinoma samples (data not shown).

### Sox11 protein expression in ovarian cancer

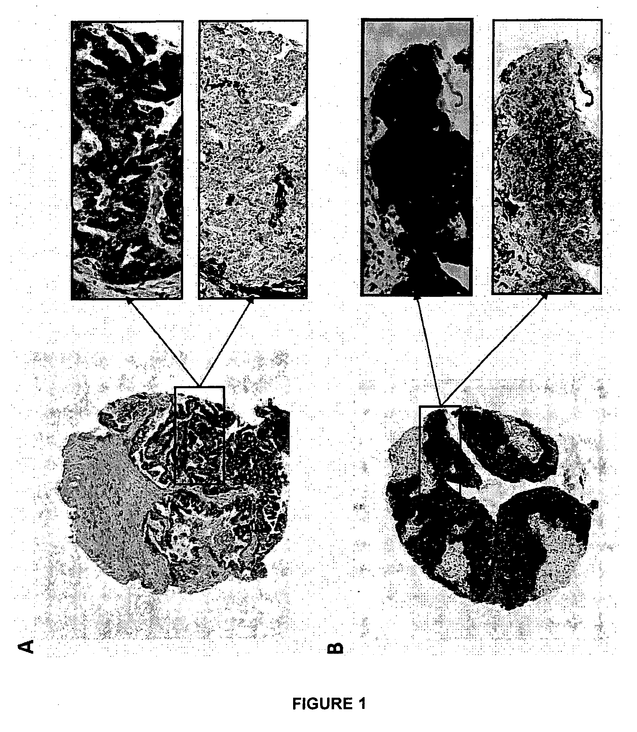

Having identified Sox11 as a gene that was overexpressed in epithelial ovarian carcinoma cells, Sox11 protein expression was examined using IHC in EOC as illustrated in Figure 1. Sox11 expression was seen exclusively in tumour epithelium and IHC signal was evident in both the nucleus and the cytoplasm. Nuclear expression of Sox11 was present only when accompanied by cytoplasmic signal, whereas a proportion (49%) of tumours did demonstrate cytoplasmic expression in the absence of nuclear signal (Fig. 1).

### Quantitative determination of Sox11 expression as determined by image analysis

Quantitative determination of Sox11 expression was then ascertained, using an image analysis approach, in particular via the use of a commercial colour deconvolution algorithm (Aperio). A pseudo-colour "mark-up" image was generated as an algorithm result, thus allowing confirmation that the algorithm was accurately identifying epithelial and stromal pixels (Fig. 1). A full description of the algorithm was recently published (Brennan DJ, Rexhepaj E, O'Brien SL, et al. Altered Cytoplasmic-to-Nuclear Ratio of Survivin Is a Prognostic Indicator in Breast Cancer. Clinical cancer research 2008;14:2681-9).

The algorithm was used to calculate a total intensity (TI) for Sox11 for the each core. There was a strong correlation between quadruplicate cores from individual tumours for TI (Spearman's Rho = 0.858, p < 0.001), indicating that Sox11 has a homogenous pattern of expression in ovarian cancer and is suitable for TMA based analysis. As tumours were arrayed in quadruplicate, the median value for each tumour was used for further analysis. The algorithm accurately distinguished between nuclear and cytoplasmic staining in all cores, as confirmed by a histopathologist.

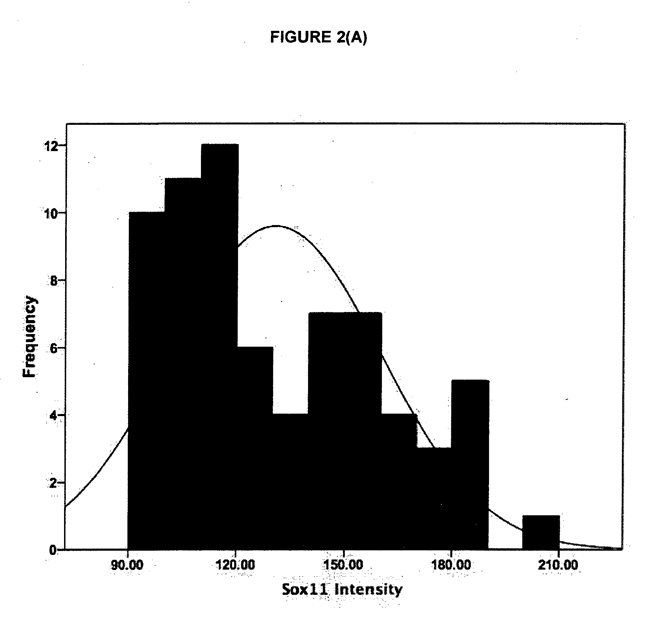

A histogram of Sox11 image analysis data for the entire cohort is shown in Figure 2a. Using this histogram, the tumours were placed into three categories - high, medium and low level of Sox11 expression, as determined by image analysis. Based on image analysis categorization, 20% (n = 17) of tumours were classified as having high levels 43% (n = 35) medium levels and 29% (n = 24) low/negative levels of Sox11 expression, as determined by image analysis. Tumours in the high expression group all showed expression of nuclear and cytoplasmic Sox11, whilst those in the medium group generally exhibited only cytoplasmic Sox11. No association was found between Sox11 expression and age, grade or stage of disease.

### Associations between Sox11 expression as determined by automated image analysis and survival

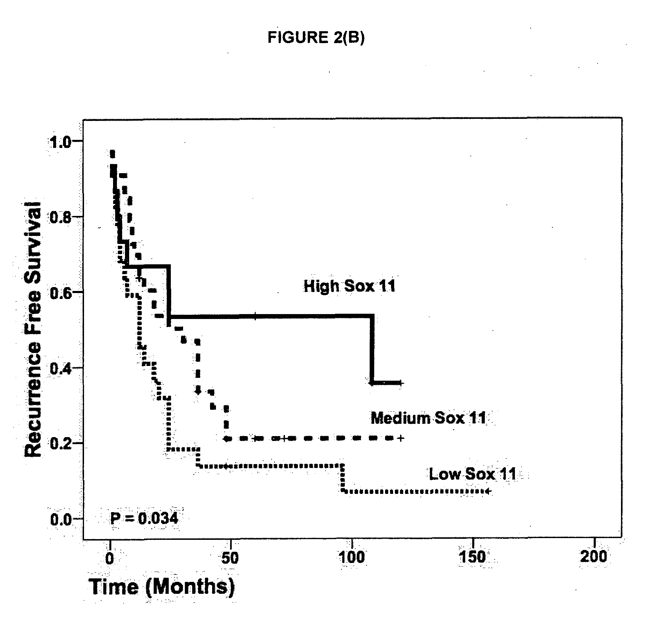

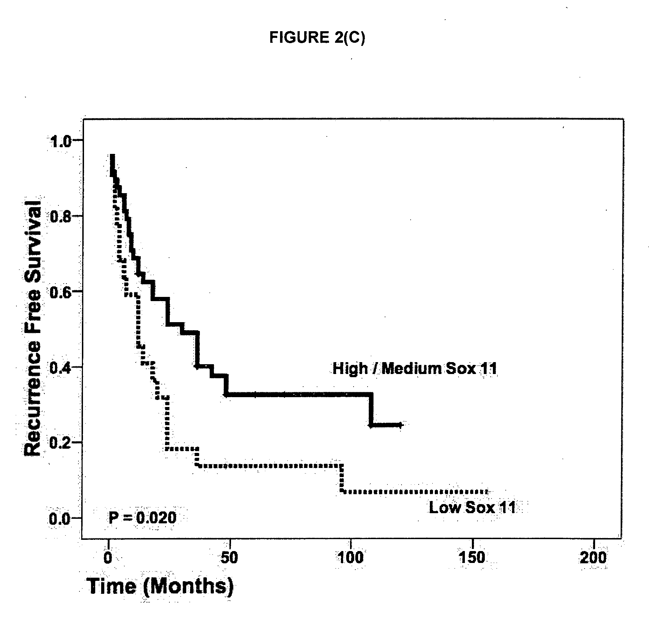

Kaplan Meier analysis of RFS based on the expression of Sox11 revealed a stepwise decrease in RFS between the high, medium and low groups (p = 0.033) (Fig. 2b). Further subset analysis revealed a markedly reduced RFS in patients with low levels of Sox11 expression, as determined by image analysis compared to high and medium Sox11 expressers (p = 0.02) (Fig. 2c). We proceeded to perform a multivariate Cox regression analysis of RFS, which revealed that Sox11 expression was an independent predictor of RFS when compared to stage and grade (HR = 0.56, 95% CI = 0.319 - 0.997, p = 0.049).

### Discussion

In this study we combined a meta-analysis of transcriptomic data, TMAs and automated image analysis to identify and validate Sox11 as a prognostic biomarker in epithelial ovarian carcinoma (EOC).This study is the first to describe the relationship between Sox11 expression and prognosis in EOC. Having previously identified Sox11 as a new diagnostic marker in MCL (Ek S, Dictor M, Jerkeman M, Jirstrom K, and Borrebaeck CA. Nuclear expression of the non B-cell lineage Sox11 transcription factor identifies mantle cell lymphoma. Blood 2008;111:800-5), we proceeded to use the IST database containing data from a meta-analysis of 14 095 samples analyzed, using the Affymetrix gene expression microarrays to profile the expression of Sox11 mRNA in EOC cells.

We then proceeded to use TMAs and a quantitative automated analysis of IHC to evaluate Sox11 protein expression in EOC in relation to recurrence free survival. This revealed that epithelial-specific Sox11 expression in both the nuclear and cytoplasmic compartments. Increased levels of Sox11, particularly nuclear Sox11, was associated with an increased RFS and Cox regression multivariate analysis revealed Sox11 was an independent predictor of RFS when controlling for grade and stage (see Table 2).

Sox11 plays an important role in embryogenesis and tissue remodeling, and consequently is present during gastrulation and early post-gastrulation development throughout the embryo (Hargrave M, Wright E, Kun J, et al. Expression of the Sox11 gene in mouse embryos suggests roles in neuronal maturation and epithelio-mesenchymal induction. Developmental dynamics 1997;210:79-86; Sock E, Rettig SD, Enderich J, et al. Gene targeting reveals a widespread role for the high-mobility-group transcription factor Sox11 in tissue remodeling. Molecular and cellular biology 2004;24:6635-44). Later during development, Sox11 is prominently expressed in the developing nervous system and at many sites throughout the embryo where epithelial-mesenchymal interactions occur (Hargrave M, Wright E, Kun J, et al. Expression of the Sox11 gene in mouse embryos suggests roles in neuronal maturation and epithelio-mesenchymal induction. Developmental dynamics 1997;210:79-86). At sites of such epithelial-mesenchymal interactions, Sox11 can be found in the mesenchymal or epithelial compartment, and it has been postulated to be involved in inductive remodelling (Hargrave M, et al., 1997*supra*.). Sox11 expression in most tissues is transient and as a consequence, little Sox11 expression has been found in terminally differentiated adult tissues, in contrast to its widespread expression during embryogenesis (Sock E, Rettig SD, Enderich J, et al. Gene targeting reveals a widespread role for the high-mobility-group transcription factor Sox11 in tissue remodeling. Molecular and cellular biology 2004;24:6635-44). Our findings complement these data, whereby Sox11 expression was absent in normal tissue. The role played by Sox11 in tumourogenesis remains to be fully elucidated. As mentioned previously, a marked upregulation of Sox11 mRNA was evident in EOC cells. The exact functional role of Sox11 in adult tissues is not fully understood, although the Sox proteins appear to play a dual role (i) DNA binding and (ii) transcriptional partner selection, which may permit selective recruitment of individual Sox proteins to specific genes (Ek S, Dictor M, Jerkeman M, Jirstrom K, and Borrebaeck CA. Nuclear expression of the non B-cell lineage Sox11 transcription factor identifies mantle cell lymphoma. Blood 2008;111:800-5). Whilst a number of studies have described Sox11 expression in gliomas (Weigle B, Ebner R, Temme A, et al. Highly specific overexpression of the transcription factor SOX11 in human malignant gliomas. Oncology reports 2005;13:139-44), neuroblastomas (Lee CJ, Appleby VJ, Orme AT, Chan WI, and Scotting PJ. Differential expression of SOX4 and SOX11 in medulloblastoma. Journal of neuro-oncology 2002;57:201-14) and MCL (Ek S, Dictor M, Jerkeman M, Jirstrom K, and Borrebaeck CA. Nuclear expression of the non B-cell lineage Sox11 transcription factor identifies mantle cell lymphoma. Blood 2008;111:800-5), its functional role in these tumours is not completely understood.

In summary, this is the first description of the differential expression of Sox11 in EOC. Our findings demonstrate that Sox11 is an independent predictor of improved RFS in EOC.

**Table 1. Patient and tumour characteristics**

| **Age** | |
|---|---|
| Median (Range) | 52 (31-77) |
| | |

| **Histology** | |
|---|---|
| Serous | 50 |
| Mucinous | 4 |
| Endometroid | 17 |
| Clear cell | 1 |
| Other | 4 |
| | |

| **Grade** | |
|---|---|
| Well Differentiated | 12 |
| Moderately Differentiated | 29 |
| Poorly Differentiated | 35 |
| | |

| **Stage** | |
|---|---|
| 1 | 0 |
| 2 | 21 |
| 3 | 54 |
| 4 | 1 |

**Table 2. Multivariate^{*} Cox regression analysis of RFS**

| | **HR** | **95.0% CI** | **P value** |
|---|---|---|---|
| **Sox11** | | | |
| (*highlmedium v's low)* | 0.56 | 0.319 - 0.997 | 0.049 |
| **Stage** | | | |
| *(continuous)* | 1.92 | 0.971 - 3.800 | 0.061 |
| **Grade** | | | |
| (*Well and moderately diff v's poorly* | | | |
| *Diff)* | 1.08 | 0.740 - 1.562 | 0.702 |

| | | | |
|---|---|---|---|
| *Adjusted for all other variables in the table Abbreviations: HR = Hazard ratio, 95% CI = 95% Confidence intervals | | | |

### EXAMPLE B

### Introduction

Epithelial ovarian cancer (EOC) comprises three major histological subtypes (serous, mucinous and endometrioid) and can also be subgrouped based on stage and grade. Endometrioid tumors make up about 2 to 4 percent of all ovarian tumors and most of them (about 80 percent) are malignant, representing 10 to 20 percent of all ovarian carcinomas.

### Material & Methods

Sections of high grade EOC and endometrioid EOC were stained for Sox11 and analyzed as previously described (Brennan et al., 2009, European Journal of Cancer, 45(8):1510-1517).

### Results & Discussion

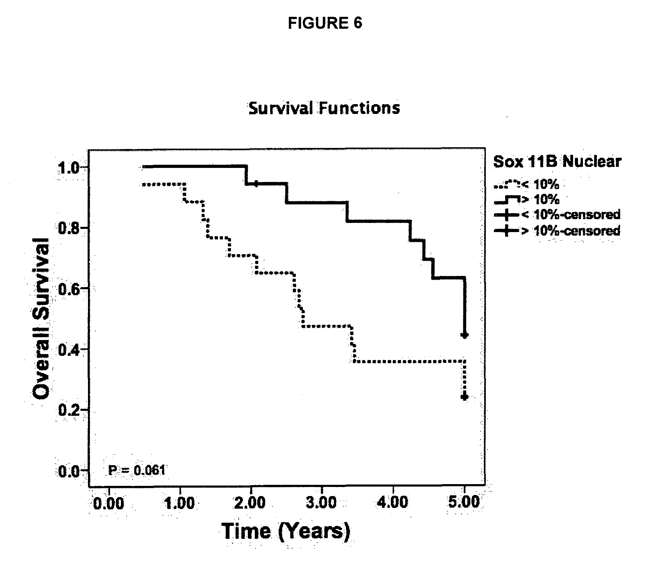

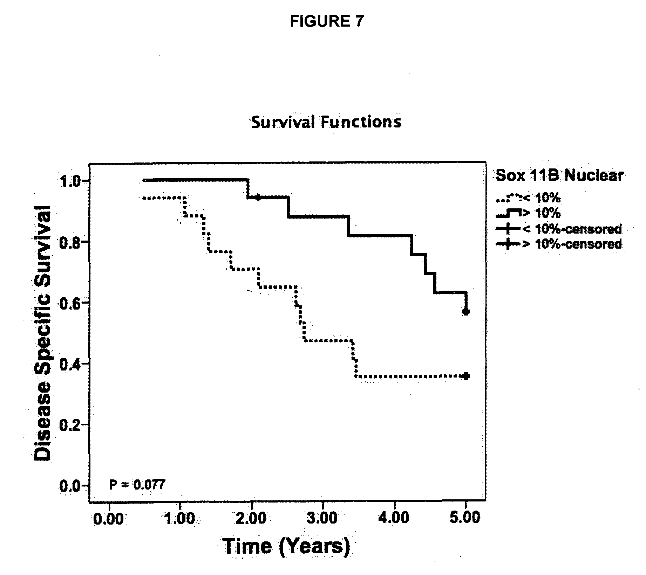

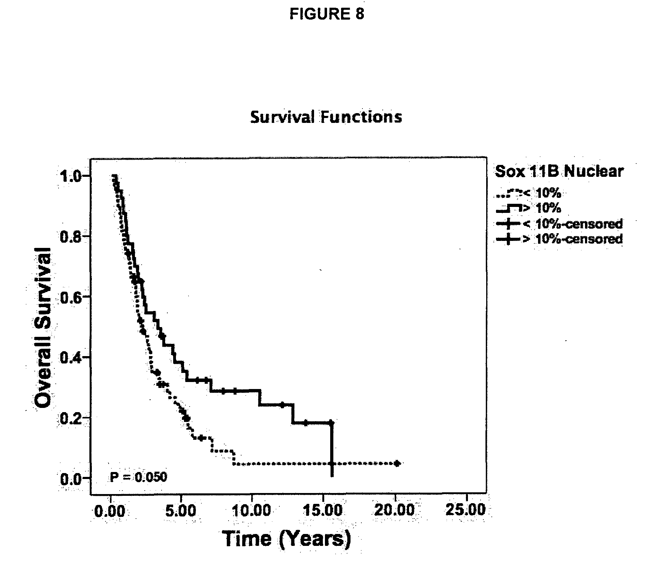

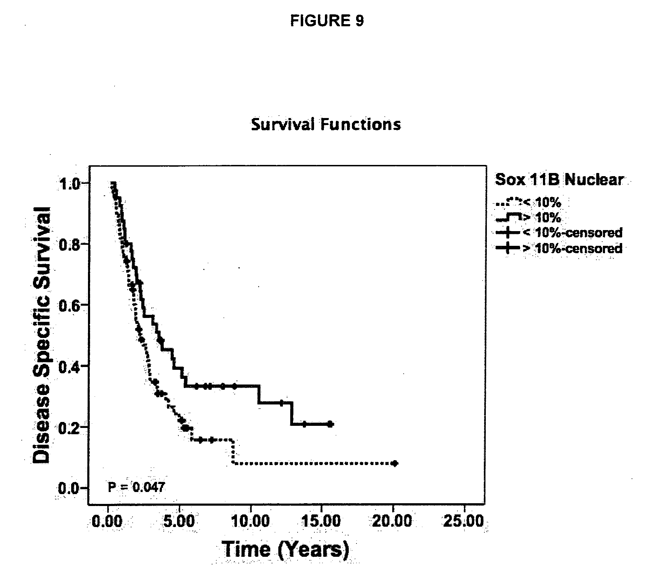

As shown in Figures 5, 6 and 7, overall and cancer specific survival can be predicted using Sox11 for endometrioid EOCs. Also, as shown in Figures 8 and 9, overall and cancer specific survival can be predicted using Sox11 for high grade EOCs.

When calculating cancer-specific survival probability only data for cancer-related deaths are used, in contrast to when calculating overall survival.

These data indicate that Sox11 can be used in both high grade EOC and endometrioid ovarian cancer to stratify patients into clinically relevant groups based on overall and cancer specific survival.

In conclusion, Sox11 is not only a useful biomarker for EOC as a group, but can be used for subgroups of patients with different clinical and/or histological features.

## Claims

1. A binding moiety which is capable of binding selectively to Sox11 protein, or to a nucleic acid molecule encoding the same, for use in diagnosing, prognosing and or detecting epithelial ovarian cancer (EOC) *in vivo.*

2. The use of a binding moiety which is capable of binding selectively to Sox11 protein, or to a nucleic acid molecule encoding the same for diagnosing, prognosing and/or detecting epithelial ovarian cancer (EOC) *in vitro.*

3. A binding moiety for use in diagnosing, prognosing and or detecting epithelial ovarian cancer (EOC) *in vivo* according to Claim 1 or a use for diagnosing, prognosing and/or detecting epithelial ovarian cancer (EOC) *in vitro* according to Claim 2, wherein the EOC belongs to a histological subtype selected from the group consisting of serous, mucinous, endometrioid and clear cell.

4. A binding moiety for use in diagnosing, prognosing and or detecting epithelial ovarian cancer (EOC) *in vivo* according to Claim 1 or 3, or a use for diagnosing, prognosing and/or detecting epithelial ovarian cancer (EOC) *in vitro* according to Claim 2 or 3 in the detection of Sox11 expression as a sole biomarker for diagnosing or prognosing epithelial ovarian cancer (EOC).

5. A binding moiety for use in diagnosing, prognosing and or detecting epithelial ovarian cancer (EOC) *in vivo* according to Claim 1, 3 or 4, or a use for diagnosing, prognosing and/or detecting epithelial ovarian cancer (EOC) *in vitro* according to Claim 2, 3 or 4 wherein the binding moiety is capable of binding selectively to Sox11 protein.

6. A binding moiety for use in diagnosing, prognosing and or detecting epithelial ovarian cancer (EOC) *in vivo* according to Claim 1, 3, 4 or 5 or a use for diagnosing, prognosing and/or detecting epithelial ovarian cancer (EOC) *in vitro* according to Claim 2, 3, 4 or 5 wherein the binding moiety is capable of binding selectively to a polypeptide comprising an amino acid sequence of SEQ ID NO: 1.

7. A binding moiety for use in diagnosing, prognosing and or detecting epithelial ovarian cancer (EOC) *in vivo* according to any one of Claims 1 or 3 to 6, or a use for diagnosing, prognosing and/or detecting epithelial ovarian cancer (EOC) *in vitro* according to any one Claims 2 to 6, wherein the binding moiety comprises or consists of an antibody or an antigen-binding fragment thereof.

8. A binding moiety for use in diagnosing, prognosing and or detecting epithelial ovarian cancer (EOC) *in vivo* according to Claim 1, 3 or 4, or a use for diagnosing, prognosing and/or detecting epithelial ovarian cancer (EOC) *in vitro* according to any one of Claims 2 to 4 wherein the binding moiety is capable of binding selectively to a nucleic acid molecule encoding Sox11 protein.

9. A binding moiety for use in diagnosing, prognosing and or detecting epithelial ovarian cancer (EOC) *in vivo* according to any one of Claims 1, 3, 4 or 8, or a use for diagnosing, prognosing and/or detecting epithelial ovarian cancer (EOC) *in vitro* according to any one of Claims 2 to 4 or 8 wherein the binding moiety is capable of binding selectively to a nucleic acid molecule encoding a polypeptide comprising an amino acid sequence of SEQ ID NO: 1.

10. A binding moiety for use in diagnosing, prognosing and or detecting epithelial ovarian cancer (EOC) *in vivo* according to any one of Claims 1 or 3 to 9 or a use for diagnosing, prognosing and/or detecting epithelial ovarian cancer (EOC) *in vitro* according to any one of Claims 2 to 9, wherein the binding moiety comprises a detectable moiety.

11. An *in vitro* method of diagnosing epithelial ovarian cancer (EOC) in an individual, the method comprising:
(a) providing a sample of epithelial ovarian cells from the individual; and
(b) determining the amount of Sox11 protein and/or mRNA in the sample of cells.
wherein the levels of Sox11 protein and/or mRNA are indicative of the individual having epithelial ovarian cancer (EOC).

12. An *in vitro* method of prognosing epithelial ovarian cancer (EOC) in an individual, the method comprising:
(a) providing a sample of epithelial ovarian cancer cells from the individual; and
(b) determining the amount of Sox11 protein and/or mRNA in the sample of cells.
wherein the levels of Sox11 protein and/or mRNA are indicative the individual having improved recurrence-free survival (RFS).

13. An *in vitro* method of detecting epithelial ovarian cancer (EOC) cells in an individual, the method comprising:
(a) providing a sample of epithelial ovarian cells from the individual; and
(b) determining the amount of Sox11 protein and/or mRNA in the sample of cells.
wherein the levels of Sox11 protein and/or mRNA are indicative of the individual having epithelial ovarian cancer (EOC) cells.

14. A binding moiety as defined in any one of claims 1 or 3 to 10 for use in a method of imaging epithelial ovarian cancer (EOC) cells in the body of an individual, the method comprising administering to the individual an effective amount of the said binding moiety.

## Patentansprüche

1. Bindungsmolekülteil, der in der Lage ist, selektiv an Sox11-Protein oder an ein Nukleinsäuremolekül, das für selbiges kodiert, zu binden, für den Gebrauch beim Diagnostizieren, Prognostizieren und/oder Erkennen von Epitheleierstockkrebs (epithelial ovarian cancer - EOC) *in vivo.*

2. Gebrauch eines Bindungsmolekülteils, der in der Lage ist, selektiv an Sox11-Protein oder an ein Nukleinsäuremolekül, das für selbiges kodiert, zu binden, zum Diagnostizieren, Prognostizieren und/oder Erkennen von Epitheleierstockkrebs (EOC) *in vitro.*

3. Bindungsmolekülteil für den Gebrauch beim Diagnostizieren, Prognostizieren und/oder Erkennen von Epitheleierstockkrebs (EOC) *in vivo* nach Anspruch 1 oder Gebrauch zum Diagnostizieren, Prognostizieren und/oder Erkennen von Epitheleierstockkrebs (EOC) *in vitro* nach Anspruch 2, wobei der EOC zu einem histologischen Subtyp gehört, ausgewählt aus der Gruppe bestehend aus seröser, muzinöser, endometrialer und klarer Zelle.

4. Bindungsmolekülteil für den Gebrauch beim Diagnostizieren, Prognostizieren und/oder Erkennen von Epitheleierstockkrebs (EOC) *in vivo* nach Anspruch 1 oder 3, oder Gebrauch zum Diagnostizieren, Prognostizieren und/oder Erkennen von Epitheleierstockkrebs (EOC) *in vitro* nach Anspruch 2 oder 3 beim Erkennen von Sox11-Expression als alleinigen Biomarker zum Diagnostizieren oder Prognostizieren von Epitheleierstockkrebs (EOC).

5. Bindungsmolekülteil für den Gebrauch beim Diagnostizieren, Prognostizieren und/oder Erkennen von Epitheleierstockkrebs (EOC) *in vivo* nach Anspruch 1, 3 oder 4 oder Gebrauch zum Diagnostizieren, Prognostizieren und/oder Erkennen von Epitheleierstockkrebs (EOC) *in vitro* nach Anspruch 2, 3 oder 4, wobei der Bindungsmolekülteil in der Lage ist, selektiv an Sox11-Protein zu binden.

6. Bindungsmolekülteil für den Gebrauch beim Diagnostizieren, Prognostizieren und/oder Erkennen von Epitheleierstockkrebs (EOC) *in vivo* nach Anspruch 1, 3, 4 oder 5 oder Gebrauch zum Diagnostizieren, Prognostizieren und/oder Erkennen von Epitheleierstockkrebs (EOC) *in vitro* nach Anspruch 2, 3, 4 oder 5, wobei der Bindungsmolekülteil in der Lage ist, selektiv an ein Polypeptid zu binden, das eine Aminosäuresequenz aus SEQ ID NO: 1 umfasst.

7. Bindungsmolekülteil für den Gebrauch beim Diagnostizieren, Prognostizieren und/oder Erkennen von Epitheleierstockkrebs (EOC) *in vivo* nach einem der Ansprüche 1 oder 3 bis 6 oder Gebrauch zum Diagnostizieren, Prognostizieren und/oder Erkennen von Epitheleierstockkrebs (EOC) *in vitro* nach einem der Ansprüche 2 bis 6, wobei der Bindungsmolekülteil einen Antikörper oder ein antigenbindendes Fragment davon umfasst oder daraus besteht.

8. Bindungsmolekülteil für den Gebrauch beim Diagnostizieren, Prognostizieren und/oder Erkennen von Epitheleierstockkrebs (EOC) *in vivo* nach Anspruch 1, 3 oder 4 oder Gebrauch zum Diagnostizieren, Prognostizieren und/oder Erkennen von Epitheleierstockkrebs (EOC) *in vitro* nach einem der Ansprüche 2 bis 4, wobei der Bindungsmolekülteil in der Lage ist, selektiv an ein Nukleinsäuremolekül, das für Sox11-Protein kodiert, zu binden.

9. Bindungsmolekülteil für den Gebrauch beim Diagnostizieren, Prognostizieren und/oder Erkennen von Epitheleierstockkrebs (EOC) *in vivo* nach Anspruch 1, 3, 4 oder 8 oder Gebrauch zum Diagnostizieren, Prognostizieren und/oder Erkennen von Epitheleierstockkrebs (EOC) *in vitro* nach Anspruch 2 bis 4 oder 8, wobei der Bindungsmolekülteil in der Lage ist, selektiv an ein Nukleinsäuremolekül zu binden, das für ein Polypeptid kodiert, welches eine Aminosäuresequenz aus SEQ ID NO: 1 umfasst.

10. Bindungsmolekülteil für den Gebrauch beim Diagnostizieren, Prognostizieren und/oder Erkennen von Epitheleierstockkrebs (EOC) *in vivo* nach einem der Ansprüche 1 oder 3 bis 9 oder Gebrauch zum Diagnostizieren, Prognostizieren und/oder Erkennen von Epitheleierstockkrebs (EOC) *in vitro* nach einem der Ansprüche 2 bis 9, wobei der Bindungsmolekülteil einen erkennbaren Molekülteil umfasst.

11. *In-vitro-Verfahren* zum Diagnostizieren von Epitheleierstockkrebs (EOC) in einem Individuum, wobei das Verfahren Folgendes umfasst:
(a) Bereitstellen einer Probe von Epitheleierstockzellen von dem Individuum; und
(b) Bestimmen der Menge von Sox11-Protein und/oder mRNA in der Zellprobe,
wobei der Gehalt an Sox11-Protein und/oder mRNA anzeigt, dass das Individuum Epitheleierstockkrebs (EOC) hat.

12. *In-vitro-Verfahren* zum Prognostizieren von Epitheleierstockkrebs (EOC) in einem Individuum, wobei das Verfahren Folgendes umfasst:
(a) Bereitstellen einer Probe von Epitheleierstockkrebszellen von dem Individuum; und
(b) Bestimmen der Menge von Sox11-Protein und/oder mRNA in der Zellprobe,
wobei der Gehalt an Sox11-Protein und/oder mRNA anzeigt, dass das Individuum verbessertes rezidivfreies Überleben (recurrence-free survival - RFS) aufweist.

13. *In-vitro-Verfahren* zum Erkennen von Epitheleierstockkrebs (EOC) in einem Individuum, wobei das Verfahren Folgendes umfasst:
(a) Bereitstellen einer Probe von Epitheleierstockzellen von dem Individuum; und
(b) Bestimmen der Menge von Sox11-Protein und/oder mRNA in der Zellprobe,
wobei der Gehalt an Sox11-Protein und/oder mRNA anzeigt, dass das Individuum Epitheleierstockkrebs(EOC)-Zellen aufweist.

14. Bindungsmolekülteil nach einem der Ansprüche 1 oder 3 bis 10 für den Gebrauch bei einem Verfahren zum Abbilden von Epitheleierstockkrebs(EOC)-Zellen in dem Körper eines Individuums, wobei das Verfahren das Verabreichen einer wirksamen Menge des Bindungsmolekülteils an das Individuum umfasst.

## Revendications

1. Fraction de liaison qui est capable de se lier sélectivement à une protéine Sox11, ou à une molécule d'acide nucléique codant celle-ci, pour une utilisation dans le diagnostic, le pronostic et/ou la détection du cancer épithélial de l'ovaire (CEO) *in vivo.*

2. Utilisation d'une fraction de liaison qui est capable de se lier sélectivement à une protéine Sox11, ou à une molécule d'acide nucléique codant celle-ci, destinée au diagnostic, au pronostic et/ou à la détection du cancer épithélial de l'ovaire (CEO) *in vitro.*

3. Fraction de liaison pour une utilisation dans le diagnostic, le pronostic et/ou la détection du cancer épithélial de l'ovaire (CEO) *in vivo* selon la revendication 1, ou utilisation destinée au diagnostic, au pronostic et/ou à la détection du cancer épithélial de l'ovaire (CEO) *in vitro* selon la revendication 2, dans laquelle le CEO appartient à un sous-type histologique choisi dans le groupe consistant en une cellule séreuse, mucineuse, endométriale ou claire.

4. Fraction de liaison pour une utilisation dans le diagnostic, le pronostic et/ou la détection du cancer épithélial de l'ovaire (CEO) *in vivo* selon la revendication 1 ou 3, ou utilisation destinée au diagnostic, au pronostic et/ou à la détection du cancer épithélial de l'ovaire (CEO) *in vitro* selon la revendication 2 ou 3 dans la détection de l'expression de Sox11 comme unique biomarqueur pour le diagnostic ou le pronostic du cancer épithélial de l'ovaire (CEO.

5. Fraction de liaison pour une utilisation dans le diagnostic, le pronostic et/ou la détection du cancer épithélial de l'ovaire (CEO) *in vivo* selon la revendication 1, 3 ou 4, ou utilisation destinée au diagnostic, au pronostic et/ou à la détection du cancer épithélial de l'ovaire (CEO) *in vitro* selon la revendication 2, 3 ou 4, dans laquelle la fraction de liaison est capable de se lier sélectivement à la protéine Sox11.

6. Fraction de liaison pour une utilisation dans le diagnostic, le pronostic et/ou la détection du cancer épithélial de l'ovaire (CEO) *in vivo* selon la revendication 1, 3, 4 ou 5, ou utilisation destinée au diagnostic, au pronostic et/ou à la détection du cancer épithélial de l'ovaire (CEO) *in vitro* selon la revendication 2, 3, 4 ou 5, dans laquelle la fraction de liaison est capable de se lier sélectivement à un polypeptide comprenant une séquence d'acides aminés de SEQ ID NO : 1.

7. Fraction de liaison pour une utilisation dans le diagnostic, le pronostic et/ou la détection du cancer épithélial de l'ovaire (CEO) *in vivo* selon l'une quelconque des revendications 1, ou 3 à 6, ou utilisation destinée au diagnostic, au pronostic et/ou à la détection du cancer épithélial de l'ovaire (CEO) *in vitro* selon l'une quelconque des revendications 2 à 6, dans laquelle la fraction de liaison comprend ou consiste en un anticorps ou un fragment se liant à l'antigène de celui-ci.

8. Fraction de liaison pour une utilisation dans le diagnostic, le pronostic et/ou la détection du cancer épithélial de l'ovaire (CEO) *in vivo* selon la revendication 1, 3 ou 4, ou utilisation destinée au diagnostic, au pronostic et/ou à la détection du cancer épithélial de l'ovaire (CEO) *in vitro* selon l'une quelconque des revendications 2 à 4, dans laquelle la fraction de liaison est capable de se lier sélectivement à une molécule d'acide nucléique codant la protéine Sox11.

9. Fraction de liaison pour une utilisation dans le diagnostic, le pronostic et/ou la détection du cancer épithélial de l'ovaire (CEO) *in vivo* selon l'une quelconque des revendications 1, 3, 4 ou 8, ou utilisation destinée au diagnostic, au pronostic et/ou à la détection du cancer épithélial de l'ovaire (CEO) *in vitro* selon l'une quelconque des revendications 2 à 4 ou 8, dans laquelle la fraction de liaison est capable de se lier sélectivement à une molécule d'acide nucléique codant un polypeptide comprenant une séquence d'acides aminés de SEQ ID NO : 1.

10. Fraction de liaison pour une utilisation dans le diagnostic, le pronostic et/ou la détection du cancer épithélial de l'ovaire (CEO) *in vivo* selon l'une quelconque des revendications 1 ou 3 à 9 ou utilisation destinée au diagnostic, au pronostic et/ou à la détection du cancer épithélial de l'ovaire (CEO) *in vitro* selon l'une quelconque des revendications 2 à 9, dans laquelle la fraction de liaison comprend une fraction détectable.

11. Procédé *in vitro* de diagnostic du cancer épithélial de l'ovaire (CEO) chez un individu, le procédé comprenant :
(a) la fourniture d'un échantillon de cellules épithéliales ovariennes provenant de l'individu ; et
(b) la détermination de la quantité de protéine Sox11 et/ou d'ARNm dans l'échantillon de cellules ;
dans lequel les taux de protéine Sox11 et/ou d'ARNm indiquent que l'individu souffre d'un cancer épithélial de l'ovaire (CEO).

12. Procédé *in vitro* de diagnostic du cancer épithélial de l'ovaire (CEO) chez un individu, le procédé comprenant :
(a) la fourniture d'un échantillon de cellules épithéliales ovariennes provenant de l'individu ; et
(b) la détermination de la quantité de protéine Sox11 et/ou d'ARNm dans l'échantillon de cellules ;
dans lequel les taux de protéine Sox11 et/ou d'ARNm indiquent que l'individu présente une survie sans rechute (SSR) améliorée.

13. Procédé *in vitro* de détection de cellules du cancer épithélial de l'ovaire (CEO) chez un individu, le procédé comprenant :
(a) la fourniture d'un échantillon de cellules épithéliales ovariennes provenant de l'individu ; et
(b) la détermination de la quantité de protéine Sox11 et/ou d'ARNm dans l'échantillon de cellules ;
dans lequel les taux de protéine Sox11 et/ou d'ARNm indiquent que l'individu est porteur de cellules du cancer épithélial de l'ovaire (CEO).

14. Fraction de liaison telle que définie selon l'une quelconque des revendications 1 ou 3 à 10 pour une utilisation dans un procédé d'imagerie de cellules du cancer épithélial de l'ovaire (CEO) dans le corps d'un individu, le procédé comprenant l'administration à l'individu d'une quantité efficace de ladite fraction de liaison.
